# EUROPEAN PATENT APPLICATION

(11) **EP 4 512 415 A1**
(43) Date of publication of application: **26.02.2025**
(21) Application number: 23196345.5
(22) Date of filing: 08.09.2023
(51) Int. Cl.: A61K 39/00, A61P 25/28, A61P 37/06, C07K 14/47, C12N 15/85, C07K 16/28

(54) **CHIMERIC ANTIGEN RECEPTOR FOR TREATMENT OF MULTIPLE SCLEROSIS OR ALZHEIMER'S DISEASE**

(30) Priority: 24.08.2023 EP 23193330
(71) Applicant: Medizinische Hochschule Hannover, 30625 Hannover (DE); Technische Universität Braunschweig, 38106 Braunschweig (DE)
(72) Inventor: Jäckel, Elmar, 30625 Hannover (DE); Hardtke-Wolenski, Matthias, Hannover (DE); Sätzler, Valerie, Hannover (DE); Riet, Tobias, Hannover (DE); Hust, Michael, 30169 Hannover (DE); Helmsing, Saskia, Braunschweig (DE)
(74) Representative: Taruttis, Stefan Georg

(57) **Abstract**

The present invention relates to a CAR and to regulatory T-cells (Treg) expressing the CAR for use in the treatment of multiple sclerosis (MS) and/or Alzheimers disease (AD). The CAR, especially Treg expressing the CAR, are suitable for suppressing adverse immune reactions occurring in MS and/or AD due to the high specificity of the CAR for myelin basic protein (MBP) or myelin oligodendrocyte glycoprotein (MOG), which are primary targets of autoimmune reactions in MS and AD.

## Description

The present invention relates to a CAR and to regulatory T-cells (Treg) expressing the CAR, especially for use in the treatment of, especially for suppression of multiple sclerosis (MS) and/or Alzheimers disease (AD). The CAR, especially Treg expressing the CAR, are suitable for suppressing adverse immune reactions occurring in MS and/or AD due to the high specificity of the CAR for myelin basic protein (MBP) or myelin oligodendrocyte glycoprotein (MOG), which are primary targets of autoimmune reactions in MS and AD. Further, Treg expressing the CAR migrate to organs and tissue affected by MS and AD, especially to the brain and/or spinal cord, allowing their use in the treatment by administration to a patient at a site spaced from the brain, e.g. administration by systemic injection, e.g. intra venous administration.

The Treg of the invention that express the CAR are suitable for use in suppressing adverse immune reactions directed against brain, especially directed against MBP or MOG, specifically for use in the treatment of MS or AD, without specificity for a foreign antigen, especially without specificity for another tissue than brain or spinal cord.

An advantage of embodiments of the CAR of the invention and of Treg expressing the CAR is that the CAR is specific for brain and spinal cord, especially for MBP or MOG, and effective both in humans and in rodents, e.g. mice and rats.

### State of the art

Fransson et al., Immunology 431-441 (2014) describe expression of a CAR containing an scFv from an anti-MOG antibody in mesenchymal stromal cells for amelioration of MS. A sequence of the antibody or of the scFv is not provided.

WO 2013/153391 A1 and WO 2021/239812 A1 describe molecules for cell surface-bound expression, which can be triggered by Rituximab to cause cell death.

### Object of the invention

It is an object of the invention to provide an agent, preferably a CAR and a regulatory T-cell (Treg), for use in the treatment of multiple sclerosis and/or of Alzheimers disease. Preferably, a Treg expressing the CAR has the ability to migrate across the blood brain barrier (BBB) into the brain and/or into the spinal cord of a patient affected with MS or AD.

### Description of the invention

The invention achieves the object by the features of the claims, and especially provides a chimeric antigen receptor (CAR) that from N-terminus to C-terminus comprises or consists of an scFv portion, which is specific for the myelin basic protein (MBP) or specific for myelin oligodendrocyte glycoprotein (MOG), especially for the human and/or for the murine and rat myelin basic protein (MBP) or myelin oligodendrocyte glycoprotein (MOG), preferably a hinge region, e.g. an IgG hinge (ΔFc IgG hinge), especially a hinge comprising IgG1 - IgG1 CH2 - IgG1 CH3 hinge, or a CD8 hinge, each of human origin or of murine origin, a transmembrane domain (TM), e.g. a CD4 TM, a CD4a TM, or a CD8 TM, and intracellular signaling domains, e.g. a CD28 signaling domain and a CD3zeta signaling domain, wherein preferably the scFv portion has a pair of complementary domain regions 3 (CDR3) of the heavy and light chain. For the MBP-specific scFv domains of the CAR, the pairs of CDR3 have an amino acid sequence which comprise or consist of amino acids No. 97..105 of SEQ ID NO: 29 (VH of scFv encoded by SEQ ID NO: 25) and amino acids No. 92..100 of SEQ ID NO: 30 (VL of SEQ NO: 25), or which comprise or consist of amino acids No. 97..111 of SEQ ID NO: 31 (VH of scFv encoded by SEQ ID NO: 27) and amino acids No. 90..100 of SEQ ID NO: 32 (VL of scFv encoded by SEQ ID NO: 27), preferably, the scFv has a heavy chain (VH) containing a CDR1 of amino acids No. 26..33 of SEQ ID NO: 29, a CDR2 of amino acids No. 51..58 of SEQ ID NO: 29 and a CDR3 of amino acids No. 97..105 of SEQ ID NO: 29 in combination with a light chain (VL) containing a CDR1 of amino acids No. 26..33 of SEQ ID NO: 30, a CDR2 of amino acids No. 51..53 of SEQ ID NO: 30 and a CDR3 of amino acids No. 92..100 of SEQ ID NO: 30, or the scFv has a heavy chain (VH) containing a CDR1 of amino acids No. 26..33 of SEQ ID NO: 31, a CDR2 of amino acids No. 51..58 of SEQ ID NO: 31 and a CDR3 of amino acids No. 97..111 of SEQ ID NO: 31 in combination with a light chain (VL) containing a CDR1 of amino acids No. 26..33 of SEQ ID NO: 32, a CDR2 of amino acids No. 51..53 of SEQ ID NO: 32 and a CDR3 of amino acids No. 90..100 of SEQ ID NO: 32.

Preferably, the scFv which is specific for MBP has a variable heavy chain (VH) comprising or consisting of an amino acid sequence of
QVQLQESGGGLVKPGGSLRLSCAAS**GFTFSDYY**MSWIRQAPGKGLEWVSY**ISSSGST I**YYADSVKGRFTISRDNAKNSLYLQMNSLRAEDTAVYYC**ARGGGDFDY**WGQGTLV TVSS (SEQ ID NO: 29, VH of SH1990-C1) and a variable light chain (VL) comprising or consisting of amino acid sequence 
NFMLTQPHSVSESPGKTVTISCTGSSGSIASSYVQWYQQRPGSAPTTVIFEDYRRPSGV
PDRFSGSVDRSSNSASLTISGLKIEDEADYYCQSYGSTDVVFGGGTKLTVL (SEQ ID
NO: 30, VL of SH1990-C1),
 or the scFv has a VH comprising or consisting of amino acid sequence
QVQLQQSGAEVKKPGASVKVSCKAS**GYTFTSYY**MHWVRQAPGQGLEWMGI**INPSG GST**SYAQKFQGRVTMTRDTSTSTVYMELSSLRSEDTAVYYC**ARESVVRGVNLSFDY** WGQGTLVTVSS (SEQ ID NO: 31, VH of SH1990-C3) and a variable light chain (VL) comprising or consisting of amino acid sequence
LPVLTQPPSASGTPGQRVTISCSGS**SSNIGSNY**AYWYQQLPGTAPKLLIY**SNN**QRPSGV PDRFSGSKSGTSASLAISGLQSEDEADYYC**AAWDDSLNGVV**FGGGTKLTVL (SEQ ID NO: 32, VL of SH1990-C3). Generally, the underlined sections from N-terminus to C-terminus designate CDR1, CDR2, CDR3.

For MOG-specific scFv domains of the CAR, the pairs of CDR3 have an amino acid sequence which comprise or consist of amino acids No. 97..111 of SEQ ID NO: 51 (VH of scFv SH1986-B2) and amino acids No. 91..100 of SEQ ID NO: 52 (VL of scFv SH1986-B2), or which comprise or consist of amino acids No. 97..115 of SEQ ID NO: 53 (VH of scFv SH1986-E3) and amino acids No. 88..98 of SEQ ID NO: 54 (VL of scFv SH1986-E3), or which comprise or consist of amino acids No. 97..113 of SEQ ID NO: 55 (VH of scFv SH1986-G11) and amino acids No. 94..102 of SEQ ID NO: 56 (VL of scFv SH1986-G11), or which comprise or consist of amino acids No. 97..108 of SEQ ID NO: 57 (VH of scFv VS002-B5) and amino acids No. 88..108 of SEQ ID NO: 58 (VL of scFv VS002-B5), or which comprise or consist of amino acids No. 99..109 of SEQ ID NO: 59 (VH of scFv VS002-C4) and amino acids No. 90..100 of SEQ ID NO: 60 (VL of scFv VS002-C4), or which comprise or consist of amino acids No. 97..107 of SEQ ID NO: 61 (VH of scFv VS002-C5) and amino acids No. 92..102 of SEQ ID NO: 62 (VL of scFv VS002-C5), or which comprise or consist of amino acids No. 97..112 of SEQ ID NO: 63 (VH of scFv VS002-G9) and amino acids No. 92..100 of SEQ ID NO: 64 (VL of scFv VS002-G9), or which comprise or consist of amino acids No. 97..109 of SEQ ID NO: 65 (VH of scFv MRU185-3-C11) and amino acids No. 88..98 of SEQ ID NO: 66 (VL of scFv MRU185-3-C11),
or which comprise or consist of amino acids No. 97..106 of SEQ ID NO: 67 (VH of scFv MRU185-3-E1) and amino acids No. 89..97 of SEQ ID NO: 68 (VL of scFv MRU185-3-E1), which are specific for MOG.

Preferably, the scFv which is specific for MOG has a variable heavy chain (VH) comprising or consisting of an amino acid sequence of 
QVQLVQSGGGVVQPGRSLRLSCAASGFTFSSYAMHWVRQAPGKGLEWVAVISYDG
SNKYYADSVKGRFTISRDNSKNTLYLQMNSLRAEDTAVYYCARDAGGDSSGPYFD
YWGQGTLVTVSS (SEQ ID NO: 51, VH of SH1986-B2)
 and a variable light chain (VL) comprising or consisting of amino acid sequence 
QSALTQPASVSGSPGQSITISCTGTSSDVGGYNFVSWYQQHPGKAPKLMIYAVSERPS
GVSNRFSGSKSGNTASLTISGLQAEDEADYYCSSYRSRDALVFGGGTKLTVL (SEQ
ID NO: 52, VL of SH1986-B2),
 or the scFv has a variable heavy chain (VH) comprising or consisting of an amino acid sequence of
QVQLVQSGGGVVQPGTSLRLSCAAS**GFTFSSYG**MHWVRQAPGKGLEWVAV**ISYDG SNK**YYADSVKGRFTISRDNSKNTLYLQMNSLRAEDTAVYYC**AKDYSYYYGSGSYF NAFDI**WGQGTMVTVSS (SEQ ID NO: 53, VH of SH1986-E3) and a variable light chain (VL) comprising or consisting of amino acid sequence 
SSELTQDPAVSVALGQTVGITCQGDSLRSSFASWYQQKPGQAPILVIYGKNNRPSGIP
DRFSGSSSGNTVSLTITGAQAEDEADYYCNSRDSSGNHLVFGGGTKLTVL (SEQ ID
NO: 54, VL of SH1986-E3),
 or the scFv has a variable heavy chain (VH) comprising or consisting of an amino acid sequence of
QVQLVQSGAEVEKPGASVKVSCKVS**GYTLTKLS**MHWVRQAPGKGLEWMGG**FDPE DGET**IYAQKFQGRVTMTEDTSTDTAYMELSSLRSEDTAVYYC**ATDILGYCSSTSCY RDY**WGQGTLVTVSS (SEQ ID NO: 55, VH of SH1986-G11) and a variable light chain (VL) comprising or consisting of amino acid sequence 
EVVLTQSPLSLPISLGQPASISCRSSQSLLHSNGYNYLDWYLQKPGQSPQLLIYLGSNR
ASGVPDRFSGSGSGTDFTLKISRVEAEDVGVYYCMQALQTPRTFGQGTKVEIK (SEQ
ID NO: 56, VL of SH1986-G11),
 or the scFv has a variable heavy chain (VH) comprising or consisting of an amino acid sequence of
QVQLQQSGAEMKKPGASVKVSCKAS**GYTFTKYY**MHWVRQAPGQGLEWMGI**IDPS GGST**SYAQSFQGRVTMTRDTSTTTVYMELTSLRSEDTAVYYC**ARGGVPGHYFNW** WGQGTLVTVSS (SEQ ID NO: 57, VH of VS002-B5) and a variable light chain (VL) comprising or consisting of amino acid sequence 
QLVLTQSSSVSVAPGKTARITCGGNNIGSKSVHWYQQKPGQAPVLVIYYDSDRPSGIP
ERFSGSNSGNTATLTISRVEAGDEADYYCQVWDSSSDHPDVVFGGGTKLTVL (SEQ
ID NO: 58, VL of VS002-B5),
 or the scFv has a variable heavy chain (VH) comprising or consisting of an amino acid sequence of
OMOLVOSGGGLVQPGGSLRLSCAAS**GFTFSDYY**MDWVROAPGKGLEWVGR**IRNK ADSYNI**DYAASVTGRFTISRDDSKKSLYLQMNSLKIEDTALYYC**ARISRFDAFDI**WG QGTMVTVSS (SEQ ID NO: 59, VH of VS002-C4) and a variable light chain (VL) comprising or consisting of amino acid sequence 
QPVLTQPPSASGTPGQRVNISCSGGSSNIGSNHVFWYQQFPGTAPRLVIYRNNQRPSG
VPDRFSGSKSGTSASLVISGLRSEDEADYYCAAWDDSLSGWVFGGGTKLTVL (SEQ
ID NO: 60, VL of VS002-C4),
 or the scFv has a variable heavy chain (VH) comprising or consisting of an amino acid sequence of
QVQLVQSGGGLVQPGGSLRLSCAAS**GFTFSSYA**MSWVRQAPGKGLEWVSA**ISGSG GST**YYADSVKGRFTISRDNSKNTLYLQMNSLRAEDTAVYYC**ARGWQLWGIDY**WG QGTLVTVSS (SEQ ID NO: 61, VH of VS002-C5) and a variable light chain (VL) comprising or consisting of amino acid sequence 
LPVLTQPHSVSGSPGKTVTISCTRSSGSIAGNYVHWYQQRPGSAPTTVIYEDNQRPSG
VPDRFSGSIDSSSNSASLTISGLKTEDEADYYCQSYDGDVQHVVFGGGTKVTVL
(SEQ ID NO: 62, VL of VS002-C5),
 or the scFv has a variable heavy chain (VH) comprising or consisting of an amino acid sequence of
QVQLVESGGGLVQPGGSLRLSCAAS**GFTFSSFG**MHWVRQAPGKGLEWVSA**ISGSG GST**YYADSVKGRFTISRDNSKNTLYLQMNSLRAEDTAVYYC**AKGAYDYGYYYYG MDV**WGQGTTVTVSS (SEQ ID NO: 63, VH of VS002-G9) and a variable light chain (VL) comprising or consisting of amino acid sequence 
NFMLTQPHSVSESPGKTVTISCTRSSGSIASNYVQWYQQRPGSAPTTVIYEDNQRPSG
VPDRFSGSIDSSSNSASLTISGLKTEDEADYYCQSSDSNIEVFGGGTKLTVL (SEQ ID
NO: 64, VL of VS002-G9),
 or the scFv has a variable heavy chain (VH) comprising or consisting of an amino acid sequence of
EVQLVQSGGGVVQPGRSLRLSCAAS**GFTFSSYA**MHWVRQAPGKGLEWVAV**ISYDG SNK**YYADSVKGRFTISRDNSKNTLYLQMNSLRAEDTAVYYC**ARGFGGATSYFDY**W GQGTLVTVSS (SEQ ID NO: 65, VH of MRU185-3-C11) and a variable light chain (VL) comprising or consisting of amino acid sequence 
SSELTQDPAVSVALGQTVRITCQGDSLRSYYASWYQQKPGRAPVLVIYGKNNRPSGI
PDRFSGSSSGNTASLTITGAQAEDEADYYCNSRDSSGNPVVFGGGTKLTVL (SEQ ID
NO: 66, VL of MRU185-3-C11),
 or the scFv has a variable heavy chain (VH) comprising or consisting of an amino acid sequence of
QVQLQESGGGVVQPGRSLRLSCAAS**GFTFSSYG**MHWVRQAPGKGLEWVAV**ISYDG SKK**YYADSVKGRFTISRDNAKNTLYLQMNSLRAEDTAVYYC**ATGNSHAFDI**WGQG TMVTVSS (SEQ ID NO: 67, VH of MRU185-3-E1) and a variable light chain (VL) comprising or consisting of amino acid sequence

DIQMTQSPSTLTASVGDRVTVTCRASQTINRWLAWYQQKPGKAPKLLIYLASSLDSG
VPSRFSGSGSGTEFTLTISSLQPDDFATYYCQQYNAYPRTFGRGTKVEIK (SEQ ID
NO: 68, VL of MRU185-3-E1).

Generally, in the above sequences, the bold and underlined sections from N-terminus to C-terminus designate CDR1, CDR2, CDR3.

For localization of the CAR, its amino acid sequence preferably comprises at its N-terminus a signal peptide directing the CAR for transmembrane localization.

Generally preferred, the signal peptide, also termed leader, which is arranged N-terminally of the scFv and the domains of the CAR which are arranged C-terminally of the scFv, e.g. a hinge, a transmembrane domain and signaling domains have sequences of human origin for use in the treatment of humans, especially in order to avoid immune reactions against these domains. Herein, domains or sections of a CAR having an amino acid sequence of human origin can be identified by a "h", and domains or sections having a murine amino acid sequence can be identified by an "m". For use in rodents, e.g. mice or rats, the signal peptide and the domains of the CAR which are arranged C-terminally of the scFv can have sequences of murine origin or, less preferred, of human origin.

Optionally, the CAR is expressed as a fusion protein with FOXP3, which for use in the treatment of a human preferably has the human amino acid sequence of FOXP3 or for use in an experimental has the autologous amino acid sequence, e.g. for use in mice has the murine amino acid sequence (Foxp3). Generally, the fusion protein from N-terminus to C-terminus may comprise or consist of the CAR and FOXP3, separated by a protease site, e.g. P2A, or consist of the CAR and optionally in addition deltaLNGFR. Suitably, the FOXP3 polypeptide encoded by a nucleic acid molecule, construct or vector as described herein may comprise or consist of the polypeptide sequence of a human FOXP3, such as UniProtKB accession Q9BZS1, or a functional fragment or variant thereof.

Generally, Treg expressing the CAR is used in the treatment of MS or AD for suppressing adverse immune reactions localized in tissue containing MBP and/or MOG, especially for use in suppressing adverse immune reactions localized in brain and spinal cord. Optionally, Treg express at least one CAR containing an scFv specific for MBP and at least one CAR containing an scFv specific for MOG.

Optionally, the CAR is expressed from an expression cassette which, under the control of an IRES, encodes a peptide label, which preferably for use in humans is deltaLNGFR (SEQ ID NO: 24, encoded by SEQID NO: 23), and for use in mice is Thy1.1 (also termed CD90.1). The peptide deltaLNGFR (SEQ ID NO: 24), in addition to presenting a surface-bound label which is not immunogenic in humans and which can be detected by an antibody, e.g. a labelled antibody, deltaLNGFR also provides a peptide label suitable for specifically triggering the deletion of cells that express the CAR, e.g. using an anti-deltaLNGFR-antibody. Preferably, Treg are genetically manipulated for expression of the CAR in combination with FOXP3, e.g. expression from one common nucleic acid, preferably from one common expression cassette, with an IRES arranged between the coding sequence encoding the CAR, a protease site, and optionally FOXP3, and the coding sequence (SEQ ID NO: 23) encoding deltaLNGFR. Genetic manipulation can be by transduction of T cells, preferably of Treg, by a viral particle, preferably a retroviral particle, especially an α-retroviral or a γ-retroviral particle, the viral particle containing an expression cassette between its 5'LTR and its 3'LTR.

In embodiments of Treg that in addition to the CAR, preferably also Foxp3, also express Thy 1.1, deletion of these Treg can be triggered specifically by an anti-Thy 1.1-antibody.

In this embodiment, the Treg are suitable for use in the treatment of MS or AD by suppressing adverse immune reactions localized in tissue containing MBP and/or MOG, especially suppressing adverse immune reactions localized in brain and spinal cord, with the additional feature of terminating the activity of the Treg, e.g. deleting the Treg, by using an antibody specific for the marker peptide, herein represented by deltaLNGFR for use in humans, or Thy1.1 for use in mice, which represents a suicide moiety for binding of an antibody.

The safety switch polypeptide provides a cell in or on which it is expressed with a suicide moiety. This is useful as a safety mechanism which allows a cell which has been administered to a subject to be deleted should the need arise, or indeed more generally, according to desire or need, for example once a cell has performed or completed its therapeutic effect.

A suicide moiety possesses an inducible capacity to lead to cellular death, or more generally to elimination or deletion of a cell. An example of a suicide moiety is a suicide protein, encoded by a suicide gene, which may be expressed in or on a cell alongside a desired transgene, in this case the CAR, which when expressed allows the cell to be deleted to turn off expression of the transgene (CAR). A suicide moiety herein is a suicide polypeptide that is a polypeptide that under permissive conditions, namely conditions that are induced or turned on, is able to cause the cell to be deleted.

The suicide moiety may be a polypeptide, or amino acid sequence, which may be activated to perform a cell-deleting activity by an activating agent which is administered to the subject, or which is active to perform a cell-deleting activity in the presence of a substrate which may be administered to a subject. In a particular embodiment, the suicide moiety may represent a target for a separate cell-deleting agent which is administered to the subject. By binding to the suicide moiety, the cell-deleting agent may be targeted to the cell to be deleted. In particular, the suicide moiety may be recognised by an antibody, and binding of the antibody to the safety switch polypeptide, when expressed on the surface of a cell, causes the cell to be eliminated, or deleted.

The suicide moiety may be HSV-TK or iCasp9. However, it is preferred for the suicide moiety to be, or to comprise, an epitope which is recognised by a cell-deleting antibody or other binding molecule capable of eliciting deletion of the cell. In such an embodiment, the safety switch polypeptide is expressed on the surface of a cell.

The term "delete" as used herein in the context of cell deletion is synonymous with "remove" or "ablate" or "eliminate". The term is used to encompass cell killing, or inhibition of cell proliferation, such that the number of cells in the subject may be reduced. 100% complete removal may be desirable but may not necessarily be achieved. Reducing the number of cells, or inhibiting their proliferation, in the subject may be sufficient to have a beneficial effect.

In particular, the suicide moiety may be a CD20 epitope which is recognised by the antibody Rituximab. Thus, in the safety switch polypeptide the suicide moiety may comprise a minimal epitope based on the epitope from CD20 that is recognised by the antibody Rituximab. Biosimilars for Rituximab are available and may be used. A person of skill in the art is readily able to use routine methods to prepare an antibody having the binding specificity of Rituximab using the available amino acid sequences therefor.

CAR-cells specific for MBP or MOG, which also express a safety switch polypeptide comprising this sequence can be selectively killed using the antibody Rituximab, or an antibody having the binding specificity of Rituximab. The safety switch polypeptide is expressed on the cell surface and when the expressed polypeptide is exposed to or contacted with Rituximab, or an antibody with the same binding specificity, death of the cell ensues.

Thus, Rituximab, or an antibody having the binding specificity thereof, may be provided for use in adoptive cell transfer (ACT) in combination with a cell of the invention. The cell or nucleic acid or vector or construct for production of the cell and the Rituximab or equivalent antibody may be provided in a kit, or as a combination product.

For example, the suicide constructs of WO2013/153391 or WO2021/239812 may be used in a cell or cell population (e.g., Treg or Treg population) as described herein.

It was found that the specificity of the scFv portion for the target antigen essentially depends on the CDR3 domains of the heavy chain and of the light chain of the scFv, and that this specificity can be maintained also in combination with other CDR1 and CDR2 domains that originate from a paratope, e.g. an scFv, which is not specific for MBP or for MOG.

Generally preferred, the Treg are immunologically compatible to the recipient, and preferably the Treg originate from T cells originating from the recipient, i.e. the Treg are derived from autologous T cells, wherein they are derived by genetic manipulation to express the CAR, preferably in combination with FOXP3, and optionally express the peptide label, which preferably is CD20 or deltaLNGFR for use in humans, or Thy1.1 for use in mice.

The signal peptide can e.g. have the amino acid sequence MDFQVQIFSFLLISASVIMSRT (SEQ ID NO: 1, murine) or MWWRLWWLLLLLLLLWPMVWA (SEQ ID NO: 2, murine), preferably MWWRLWWLLLLLLLLWPMVW (SEQ ID NO: 3, human), or MDFQVQIFSFLLISASVIMSR (SEQ ID NO: 4, human).

The hinge domain, which connects the scFv portion with the transmembrane domain, can e.g. have an amino acid sequence comprising or consisting of the IgG1 hinge, the IgG1 CH2 hinge and the IgG1 CH3 hinge, the hinge region of CD28, of Cd8α, of CD4, of CD7, of CH2CH3, of an immunoglobulin, or a part or variant thereof, preferably the CD8α hinge domain or CH2CH3 hinge domain, all of human origin or all of murine origin, e.g. VPRDGGCKPCICT VPEVS S VFIFPPKPKD VLTITLTPK VTC VVVDISKDDPEVQF S WF V DDVEVHTAQTQPREEQFNSTFRSVSELPIMHQDWLNGKEFKCRVNSAAFPAPIEKTIS KTKGRPKAPQVCTIPPPKEQMAKDKVSLTCMITDFFPEDITVEWQWNGQPAENYKNT QPIMDTDGSYFVYSKLNVQKSNWEAGNTFTCSVLHEGLHNHHTEKSLSHS PG (SEQ ID NO: 5, murine) which consists of the mIgG1 hinge, the mIgG1 CH2 hinge and the mIgG1 CH3 hinge, or the CD8 hinge of human origin or of murine origin, e.g. FSSVVPVLQKVNSTTTKPVLRTPSPVHPTGTSQPQRPEDCRPRGSVKGTGLDFACDIY (SEQ ID NO: 6, murine), or e.g. FVPVFLPAKPTTTPAPRPPTPAPTIASQPLSLRPEASRPAAGGAVHTRGLDFADIYIWAP LAGTCGVLLLSLVITLYCNHRQL (SEQ ID NO: 7, human) consisting of the hCD8 hinge and the hCD28 transmembrane domain (hCD8 TM), or the deltaFc Ig, which in the CAR has the functions of a hinge domain and a transmembrane domain, e.g. having the amino acid sequence

PAEPKSPDKTHTCPPCPAPPVAGPSVFLFPPKPKDTLMIARTPEVTCVVVDVSHEDPEV
KFNWYVDGVEVHNAKTKPREEQYNSTYRVVSVLTVLHQDWLNGKEYK
CKVSNKALPAPIEKTISKAKGQPREPQVYTLPPSRDELTKNQVSLTCLVKGFYPSDIAV
EWESNGQPENNYKTTPPVLDSDGSFFLYSKLTVDKSRWQQGNVFSCSV
MHEALHNHYTQKSLSLSPGKK (SEQ ID NO: 8, human).

A transmembrane domain may be selected from the transmembrane domains of CD28, of ICOS, of CD8α, of CD4, of CD134 (OX40), of CD137 (4-1BB), of CD3 zeta, of CD45, of CD9, of CD16, of CD22, of CD33, of CD64, of CD80, of CD86, of CD154, of CH2CH3, or a part or variant thereof. Preferably, the CAR comprises a transmembrane domain of CD8α or of CH2CH3. As examples, the transmembrane domain (TM) may be the CD4 TM, e.g. VFLACVLGGSFGFLGFLGLCILCCV (SEQ ID NO: 9, murine) or its human equivalent, or the CD8a transmembrane domain CD8a TM, e.g. IWAPLAGICVALLLSLIITLICYHR (SEQ ID NO: 10, murine) or its human equivalent.

Preferably, the intracellular signaling domains comprise or consist of, from N-terminus to C-terminus, the CD28 signaling domain (CD28 ICD) and the CD3zeta signaling domain (CD3z ICD). The CD28 ICD can e.g. have the amino acid sequence NSRRNRLLQSDYMNMTPRRPGLTRKPYQPYAPARDFAAYRPL (SEQ ID NO: 11, murine) and the CD3z ICD can e.g. have the amino acid sequence RAKFSRSAETAANLQDPNQLYNELNLGRREEYDVLEKKRARDPEMGGKQQRRRNPQ EGVYNALQKDKMAEAYSEIGTKGERRRGKGHDGLYQGLSTATKDTYDA LHMQTLAPR (SEQ ID NO: 12, murine), or the CD28 ICD can e.g. have the amino acid sequence WTNSRRNRLLQSDYMNMTPRRPGLTRKPYQPYAPARDFAAYRP (SEQ ID NO: 13, murine), the CD3z ICD can e.g. have the amino acid sequence

RAKFSRSAETAANLQDPNQLYNELNLGRREEYDVLEKKRARDPEMGGKQQRRRNPQ
EGVYNALQKDKMAEAYSEIGTKGERRRGKGHDGLYQGLSTATKDTYDALHMQTLA
PR (SEQ ID NO: 14, murine).

The human CD28 ICD can e.g. have the amino acid sequence
WVRSKRSRLLHSDYMNMTPRRPGPTRKHYQPYAPPRDFAAYRS (SEQ ID NO: 15, human), the human CD3z can have the amino acid sequence
RVKFSRSADAPAYQQGQNQLYNELNLGRREEYDVLDKRRGRDPEMGGKPRRKNPQ EGLYNELQKDKMAEAYSEIGMKGERRRGKGHDGLYQGLSTATKDTYDA LHMQALPPRSSR (SEQ ID NO: 16, human), or the human CD28 ICD can have the amino acid sequence
DPKFWVLVVVGGVLACYSLLVTVAFIIFWVRSKRSRLLHSDYMNMTPRRPGPTRKH YQAYAAARDFAAYRSL (SEQ ID NO: 17, human), and the human CD28 ICD can have the amino acid sequence

RVKFSRSADAPAYQQGQNQLYNELNLGRREEYDVLDKRRGRDPEMGGKPRRKNPQ
EGLYNELQKDKMAEAYSEIGMKGERRRGKGHDGLYQGLSTATKDTYDA
LHMQALPPR (SEQ ID NO: 18, human).

The CAR preferably has an intracellular domain (ICD) comprising or consisting of one or more intracellular signaling domains selected from the group consisting of the CD3zeta signaling domain or any of its homologs, the signaling domain of a CD3 polypeptide, of a syk family tyrosine kinase, of a src family tyrosine kinase, of CD2, of CD5, and of CD8, or a signaling domain of a part or variant thereof. Preferably, the CAR comprises the CD3 zeta signaling domain. Generally preferred, the CAR comprises or consists of a human signal peptide, an scFv, human a CD28 hinge and a human CD28 transmembrane domain, a human CD28 ICD and a human CD3z ICD, or the CAR comprises or consists of a human signal peptide, an scFv, a human delta Fc Ig as a hinge and transmembrane domain, a human CD28 ICD and a human CD3z ICD, especially for use in the treatment of adverse immune reactions directed against the liver in a human.

The CAR may comprise one or more co-stimulatory domains. The one or more co-stimulatory domains may be selected from the intracellular domains of CD28, of ICOS, of CD134 (OX40), of CD137 (4-1BB), of CD27, or of TNFRSF25, or of a part or variant thereof. Preferably, the CAR may comprise a CD28 co-stimulatory domain. The one or more co-stimulatory domain can be arranged C-terminally to the transmembrane domain.

The CAR, especially the endodomains of the CAR which are arranged C-terminally to the transmembrane domain, may comprise one or more intracellular signaling domains selected from the group consisting of the CD3 zeta signaling domain or any of its homologs, a CD3 polypeptide signaling domain, a syk family tyrosine kinase signaling domain, a src family tyrosine kinase signaling domain, CD2 signaling domain, CD5 signaling domain, and CD8 signaling domain, or a part or variant thereof. Preferably, the CAR may comprise the CD3zeta signaling domain.

The CAR may comprise one or more co-stimulatory domains, e.g. arranged C-terminally to the transmembrane domain of the CAR. The one or more co-stimulatory domains may be selected from the intracellular domains of CD28, of ICOS, of CD134 (OX40), of CD137 (4-1BB), of CD27, or of TNFRSF25, or a part or variant thereof. Preferably, the CAR may comprise a CD28 co-stimulatory domain.

The CAR preferably comprises or consists of a signal peptide, an scFv, a hinge comprising Ig G1 hinge, Ig G1 CH2 hinge and Ig G1 CH3 hinge, a CD4 transmembrane domain, a CD28 ICD and a CD3zeta ICD, or the CAR comprises or consists of a signal peptide, an scFv, a CD8 hinge, a CD8a transmembrane domain, a CD28 ICD and a CD3z ICD, for use in humans in each case preferably all of human origin, for use in mice preferably all of murine origin. The CAR is preferably encoded as a fusion protein, in which C-terminally to the CAR, a protease site, e.g. P2A, and FOXP3 are linked. The P2A protease site can have the amino acid sequence ATNFSLLKQAGDVEENPGP (SEQ ID NO: 19), FOXP3 can have the amino acid sequence

MPNPRPAKPMAPSLALGPSPGVLPSWKTAPKGSELLGTRGSGGPFQGRDLRSGAHTS
SSLNPLPPSQLQLPTVPLVMVAPSGARLGPSPHLQALLQDRPHFMHQL
STVDAHAQTPVLQVRPLDNPAMISLPPPSAATGVFSLKARPGLPPGINVASLEWVSRE
PALLCTFPRSGTPRKDSNLLAAPQGSYPLLANGVCKWPGCEKVFEEPEE
FLKHCQADHLLDEKGKAQCLLQREVVQSLEQQLELEKEKLGAMQAHLAGKMALAK
APSVASMDKSSCCIVATSTQGSVLPAWSAPREAPDGGLFAVRRHLWGSH

GNSSFPEFFHNMDYFKYHNMRPPFTYATLIRWAILEAPERQRTLNEIYHWFTRMFAY
FRNHPATWKNAIRHNLSLHKCFVRVESEKGAVWTVDEFEFRKKRSQRP
NKCSNPCP (SEQ ID NO: 20, murine) or

ATNFSLLKQAGDVEENPGPSMPNPRPAKPMAPSLALGPSPGVLPSWKTAPKGSELLG
TRGSGGPFQGRDLRSGAHTSSSLNPLPPSQLQLPTVPLVMVAPSGARLGPSPHLQALL
QDRPHFMHQLSTVDAHAQTPVLQVRPLDNPAMISLPPPSAATGVFSLKARPGLPPGI
NVASLEWVSREPALLCTFPRSGTPRKDSNLLAAPQGSYPLLANGVCKWPGCEKVFEE
PEEFLKHCQADHLLDEKGKAQCLLQREVVQSLEQQLELEKEKLGAMQAHLAGKMA
LAKAPSVASMDKSSCCIVATSTQGSVLPAWSAPREAPDGGLFAVRRHLWGSHGNSSF
PEFFHNMDYFKYHNMRPPFTYATLIRWAILEAPERQRTLNEIYHWFTRMFAYFRNHP
ATWKNAIRHNLSLHKCFVRVESEKGAVWTVDEFEFRKKRSQRPNKCSNPCP (SEQ ID
NO: 21, murine).

Preferably FOXP3 (human) can have an amino acid sequence comprising or consisting of SEQ ID NO: 22.

The optional marker deltaLNGFR or CD20 for use in humans, or Thy1.1 for use in mice, can e.g. be encoded by a nucleic acid sequence that is connected in 3' to the nucleic acid sequence encoding the CAR or preferably a fusion protein of the CAR and FOXP3 by an interstitial IRES. The expression of FOXP3 in combination with expression of the CAR maintains the suppressor activity of regulatory T cells (Treg).

The CAR is preferably expressed in regulatory T cells, which are characterized by expressing the markers CD4⁺CD25⁺CD127^{low}. Preferably, the regulatory T cells are immunologically compatible with the human to be treated, more preferably the regulatory T cells originate from the human to be treated who, after genetic manipulation of the T cells such that they express the CAR, can receive the T cells expressing the CAR.

The scFv domain of the CAR molecules of the invention were generated by phage display using a human naive phage display library. A cell panning approach was performed by panning against recombinant murine or human MBP (databank entry NCBI gene ID: 4155) or MOG (databank entry NCBI gene ID: 4336). The phage display library or binding scFv of consecutive panning round were incubated with either murine and/or human MBP or murine and/or human MOG, immobilized for selecting phage displaying scFv. From eluted phage, the scFv were amplified and used for a next panning round. Panning was conducted for 3 consecutive rounds. After the last panning round, individual scFv were initially screened first for recognition of MBP or MOG. For screening, soluble scFv, provided with a His-Tag and a Myc-Tag were produced in E. coli.

Cloning of murine and human scFvs into murine and human retroviral vectors, respectively, was performed to generate CARs with anti-MBP and/or anti-MOG specificity. The CARs either had a murine CD8α hinge and transmembrane domain (short hinge) or a murine IgG hinge domain and CD4 transmembrane domain (long hinge). The sequence of the CD8α hinge is shown in SEQ ID NO: 6. Retroviral vectors containing LTR flanked 2nd generation CAR scaffolds were used for cloning. Cloning was performed by digestion of vectors with Ncol/Notl restriction enzymes and subsequent ligation of the scFv encoding nucleic acid sequence into the CAR backbone. Murine CAR backbones contained an additional Foxp3 expression cassette allowing converted Tregs (cTreg) generation out of CD4+ T cells but lacked such a cassette for generation of CAR Teffs or natural Tregs (nTregs). Vectors also contained Thy1.1. (CD90.1) as a CAR expression marker.

Generally Treg are characterized, e.g. by FACS, as CD4+ (CD4 positive), CD25high, CD127low, preferably also CD154- (CD154 negative), LAP+ (latency-associated peptide positive) and/or GARP+ (glycoprotein A repetitions predominant positive). In an embodiment, Treg cells that are not antigen-specific by nature can be isolated as CD4+, CD25high, CD127low, and can optionally be genetically manipulated to express FOXP3 to generate FOXP3+ Treg cells. For antigen-specificity, these Treg cells are genetically manipulated to express an antigen-specific CAR., e.g. isolated as and transduced with a nucleic acid construct encoding FOXP3, e.g. with a nucleic acid construct encoding both the CAR of the invention and FOXP3. Treg cells that are antigen-specific by nature, herein referred to as nTreg as they are not genetically manipulated for expression of a CAR, can be defined and isolated as CD4+ (CD4 positive), CD25high, CD127low, preferably also CD154-(CD154 negative), LAP+ and/or GARP+ and FOXP3+.

Production of recombinant CAR expressing retroviral virus and transduction of T cells and cell lines:
Retroviral particles allowing for CAR transduction of T cells were produced in HEK293T cells. They were K73 ecotropic-pseudotyped for murine constructs and VSV-G pantropic-pseudotyped for human constructs, respectively. For CAR transduction, CD4+ cells were enriched from murine spleenocytes by magnetic bead separation and activated by addition of anti-CD3/anti-CD28 beads for 2 days, followed by spin-transduction with virus particles and protaminsulfate and then underwent continued cultivation for 1 - 4 days. For a CAR activation assay, NFAT-GFP reporter murine T cell hybridoma cells were transduced in an analogous manner.

### Hybridoma NFAT activation assay:

Murine T cell hybridoma cell line reporting NFAT activation by GFP expression were transduced with CAR vector as described above. After 48 h cells were cultivated for 24 h to allow for CAR activation. In a parallel approach, MBP or MOG peptide (human or murine) that was directly coated on wells of a microtiter plate or expressed on cells was used to stimulate CAR transduced hybridoma cells. Untreated wells served as control. Cells were stained with anti-Fab antibody binding to the CAR domains for detecting CAR expression. The level of CAR activation reported by GFP and CAR expression was measured by flow cytometry. Herein, this T cell hybridoma cell line is also referred to as the reporter cell line or as reporter cells, which is used in the examples, unless indicated otherwise.

The invention is now described in greater detail by way of examples and with reference to the figures, which show in
- Fig. 1 a schematic arrangement of a nucleic acid coding sequence for domains of a fusion protein of the CAR (mIg) and FOXP3 with the coding sequence for Thy 1.1 connected by an IRES,
- Fig. 2 a schematic arrangement of a nucleic acid coding sequence for domains of a fusion protein of the CAR (mCD8) and FOXP3 with the coding sequence for Thy 1.1 connected by an IRES,
- Fig. 3 a schematic arrangement of a nucleic acid coding sequence for domains of a fusion protein of the CAR (hCD8) and FOXP3 with the coding sequence for an exemplary marker protein (dLNGFR) connected by an IRES,
- Fig. 4 a schematic arrangement of a nucleic acid coding sequence for domains of a fusion protein of the CAR (hIg) with the coding sequence for an exemplary marker protein (dLNGFR) connected by an IRES,
- Fig. 5A and Fig. 5B show flow cytometry results for native T cell hybridoma cells (untransduced) without stimulation (PBS) and with plate-bound human MBP (hMBP),
- Fig. 5C and 5D flow cytometry results for T cell hybridoma cells expressing a CAR of the invention without stimulation (PBS) and with plate-bound human MBP (hMBP),
- Fig. 5E and 5F flow cytometry results for T cell hybridoma cells expressing a CAR of the invention without stimulation (PBS) and with plate-bound human MBP (hMBP),
- Fig. 6A and 6B flow cytometry results for activation of control T cells,
- Fig. 6C and 6D flow cytometry results for activation of T cells expressing the CAR,
- Fig. 6E and 6F flow cytometry results for T cells expressing a CAR of the invention,
- Fig. 7A and Fig. 7B flow cytometry results for control Treg, and Fig. 7C-J show flow cytometry results for Treg expressing a CAR of the invention,
- Fig. 8A flow cytometry results for Treg expressing a CAR of the invention in different locations of an experimental animal, and
- Fig. 8B flow cytometry results for Treg expressing a CAR of the invention in brain tissue of three experimental animals,
- Fig. 8C a bar graph for percentage of T-cells expressing a CAR of the invention detected as Thy 1.1+ from the CD4+ population in the indicated tissues,
- Fig. 8D schematically the deletion of a FoxP3 encoding section from an expression vector encoding a CAR,
- Fig. 9A flow cytometry results for control Treg expressing a non-specific CAR (SH1319-D4, specific for PE), and for Treg expressing a CAR of the invention in different lymph nodes,
- Fig. 9B flow cytometry results for Treg expressing a control CAR specific for PE or expressing a CAR of the invention in brain of three experimental animals,
- Fig. 9C a bar graph for presence of T-cells expressing a control CAR specific for PE and or T-cells expressing a CAR of the invention in different locations of experimental animals,
- Fig. 10A a schematic of the induction of an MS disease model,
- Fig. 10B health scores of experimental animals in the MS disease model,
- Fig. 10C, 10D, 10E health scores of experimental animals during the MS disease model,
- Fig. 10F Rotarod performance data of experimental animals during the MS disease model,
- Fig. 10G, 10H, 10I health scores of experimental animals during the MS disease model,
- Fig. 11A flow cytometry results for Treg expressing a CAR of the invention in different organs of an experimental animal,
- Fig. 11B a bar graph of the relative presence of Treg expressing a CAR of the invention in different organs of an experimental animal,
- Fig. 12A-L flow cytometry results for activation of Treg expressing a CAR of the invention,
- Fig. 13 flow cytometry results for activation of T-cells expressing a CAR of the invention,
- Fig. 14 flow cytometry results for activation of T-cells expressing a CAR of the invention,
- Fig. 15A a schematic of a disease model for MS,
- Fig. 15B flow cytometry results for activation of Tregs expressing a CAR of the invention in different organs,
- Fig. 16 flow cytometry results for activation of T-cells expressing a CAR of the invention in different organs of experimental animals.

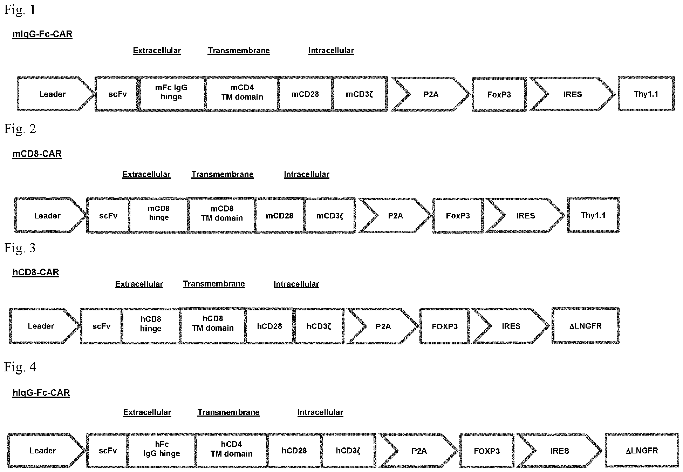

In the examples, the following CARs are used:
Signal peptide (SEQ ID NO: 1) - scFv-hinge of Ig G1 and IgG1 CH2 and IgG1 CH3 (SEQ ID NO: 5) - CD4 TM (SEQ ID NO: 9) - CD28 ICD (SEQ ID NO: 11) - CD3z ICD (SEQ ID NO: 12) - P2A (SEQ ID NO: 19) - FOXP3 (SEQ ID NO: 20), of murine origin and designated murine Ig-construct (mIg), which is schematically shown in Fig. 1,
Signal peptide (SEQ ID NO: 2) - scFv - CD8 hinge (SEQ ID NO: 6) - CD8a TM (SEQ ID NO: 10) - CD28 ICD (SEQ ID NO: 13) - CD3z ICD (SEQ ID NO: 14) - P2A - FOXP3 (SEQ ID NO: 21), of murine origin and designated murine CD8 construct (mCD8), which is schematically shown in Fig. 2,
Signal peptide (SEQ ID NO: 3) - scFv - CD8 hinge and CD8 TM (SEQ ID NO: 7) - CD28 ICD (SEQ ID NO: 15) - CD3z ICD (SEQ ID NO: 16) - P2A - FOXP3, of human origin and designated human CD8 construct (hCD8), which is schematically shown in Fig. 3,
Signal peptide (SEQ ID NO: 4) - scFv - deltaFc Ig (SEQ ID NO: 8) as hinge and transmembrane domain - CD28 ICD (SEQ ID NO: 17) - CD3z ICD (SEQ ID NO: 18), of human origin and designated human Ig construct (hIg), which is schematically shown in Fig. 4, with the specific scFv attached to the designation of the construct. Accordingly, the designations as of human origin or of murine origin generally only refer to the functional elements and domains except for the scFv, and independent the scFv.

The scFv sections have been generated by selection of scFv fragments contained in a phage display library according to affinity to immobilized MBP.

### Example 1: Expression of MBP-specific CAR and marker protein and MBP-specific activation of T-cells

Several CARs, containing one of the scFv encoded by SEQ ID NO: 25 (SH1990-C1), having the amino acid sequence of SEQ ID NO: 26, and SEQ ID NO: 27 (SH1990-C3), having the amino acid sequence of SEQ ID NO: 28, were expressed in T-cell hybridoma cells that were transduced by nucleic acid constructs containing expression cassettes for one of the CARs. Each CAR had the structure as schematically shown in Fig. 1 or Fig. 2 encoding the CAR containing a murine IgG (mFc IgG) as a hinge and a murine CD4 transmembrane domain (mCD4 TM domain, or murine CD8 (mCD8) as a hinge and a murine CD8 transmembrane domain (mCD8 TM) as a fusion protein with P2A and murine Foxp3 (mFoxp3) and with a separate internal ribosome entry site (IRES) encoding Thy 1.1, or the structure as schematically shown in Fig. 3 or Fig. 4 encoding the CAR containing human CD8 (hCD8 hinge) as a hinge and human CD8 transmembrane domain (hCD8 TM domain), or human IgG as a hinge (hFc IgG hinge) and a human CD4 transmembrane domain (hCD4 TM) as a fusion protein with P2A and human FOXP3 and with a separate internal ribosome entry site (IRES) encoding deltaLNGFR (ΔLNGFR, SEQ ID NO: 24). As an intracellular signaling domain, all CARs contained the CD28 signaling domain directly fused to the CD3zeta (CD3ζ) signaling domain, respectively murine or human.

For analysis, cells that were transduced with a nucleic acid construct encoding one of the CARs were contacted with fluorescence-labelled anti-human-IgG-antibody for detecting the CAR and fluorescence-labelled anti-Thy1.1 antibody or with anti-ΔLNGFR-antibody in the case of ΔLNGFR of this label. It was found that the CAR as detected by the anti-human IgG-antibody was expressed in equimolar amounts with Thy1.1 or ΔLNGFR, respectively.

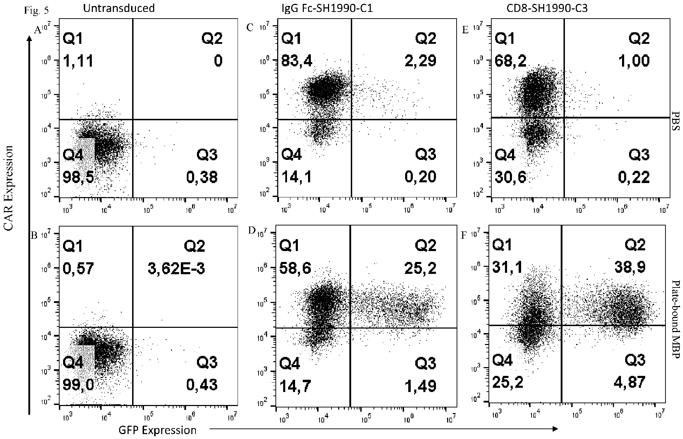

Generally, in Fig. 5 to Fig. 8 expression of the CAR (Y-axis) is detected by anti-Thy 1. 1-antibody or anti- ΔLNGFR-antibody, and activation of the T cells is detected as expression of GFP (X-axis).

Fig. 5 depicts the signals detected by flow cytometry, showing in Fig. 5A and 5B for non-transduced cells without stimulation, i.e. PBS in Fig. 5A, and with stimulation by plate-bound MBP (Fig. 5B) that cells which are not activated and which do not express a CAR of the invention are detected in quadrant 4 (Q4), i.e. PBS only or plate-bound human MBP (Plate-bound hMBP) as background control. Fig. 5C and Fig. 5D show T-cell hybridoma cells expressing the CAR having a structure according to Fig. 1 or Fig. 4 containing an scFv of SEQ ID NO: 27 with a CD8 hinge and CD8 TM domain (CD8-SH1990-C1) and Fig. 5 E and Fig. 5F expressing a CAR having a structure according to Fig. 2 or Fig. 3 containing an scFv of SEQ ID NO: 29 with an IgG hinge and CD4 TM domain (IgGFc-SH1990-C3), which without stimulation (PBS, Fig. 5C Q2 2.29%, Fig. 5E Q2 1%), are detected in quadrant 1 (Q1) and also essentially do not show auto-reactivity (quadrant 2, Q2) and which with stimulation (Plate-bound hMBP, Fig. 5D Q2 25.2%, Fig. 5F Q2 38.9%) show high expression and activation (Q2). These results show that both the embodiment of the CAR containing an IgG hinge with the CD4 TM domain and the embodiment of the CAR containing a CD8 hinge with the CD8 TM domain are effectively expressed in T cells and are specifically activated in response to presence of MBP, even plate-bound, for each of the scFv.

In relation to the cells without CAR (untransduced) detected in Fig. 5A and Fig. 5B as background, Fig. 5C shows cells expressing the CAR (83.4% in Q1) and CAR-expressing cells which are activated at only 2.29% (Q2) as unspecific background and in Fig. 5E of 1.00%, indicating absence of auto-reactivity. Fig. 5D shows that stimulation by human MBP (hMBP) results in a target antigen-specific activation of 25.2%, for the embodiment of Fig. 5F in a target-specific activation of 38.9%.

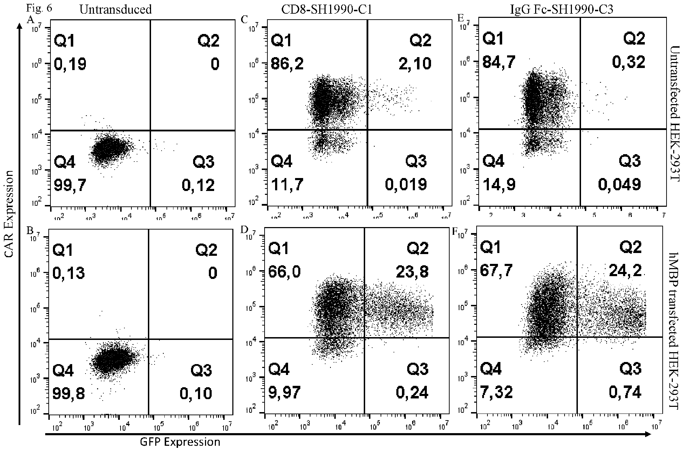

The hybridoma T-cells expressing a CAR of the invention were also target antigen-specifically activated by HEK-293 T-cells expressing hMBP. Fig. 6A and 6B show hybridoma T-cells devoid of the CAR (untransduced) in presence of HEK-293 T-cells (HEK-293T) which do not express MBP (Untransfected) as very low to absent unspecific background. Fig. 6C and 6D for the embodiment CD8-SH1990-C1 of the CAR show expression of the CAR (Q1 86.2%) CAR-expressing cells with activated background (Q2 2.10%) and in Fig. 6D specific activation of CAR-expressing cells (Q2 23.8%), both of which are comparable to background and activation with plate-bound target antigen of Fig. 5C and Fig. 5D. For the embodiment of IgGFc-SH1990-C3 of the CAR, Fig. 6E shows high expression of the CAR (Q1 84.7%) and very low background non-specific activation by non-stimulating HEK-293 T-cells (Q2 0.32), and Fig 6F for stimulation by HEK-293 T-cells expressing the target antigen hMBP shows that also this scFv induces target antigen-specific activation (Q2 24.2%, CAR expression only in Q1 67.7%) of T-cells expressing the CAR.

Further, these results show that both the embodiment of the CAR containing the CD8 hinge with the CD8 TM domain (generally indicated by CD8 in the designation of a CAR) and the embodiment of the CAR containing the ΔFcIgG hinge with the CD4 TM domain (generally indicated by IgGFc in the designation of a CAR) when expressed in a Treg result in a MBP-specific activation of the Treg.

Further, the results show that each of these scFv embodiments is specific for human MBP.

Fig. 6 shows specific activation of hybridoma T-cells by HEK-293T cells without target antigen (Figs. 6A, 6C, 6E, Untransfected HEK-293T as control) and by HEK-293T cells expressing the target antigen MBP (Figs. 6B, 6D, 6F, hMBP transfected HEK-293T). Figs. 6 A and 6B for hybridoma T-cells without a CAR (Untransduced) show no CAR expression (Q1 0.19% and 0.13%), whereas hybridoma T-cells transduced for expression of an anti-hMBP-CAR in the presence of control HEK-293T cells express the CAR (Fig. 6C, CD8-SH1990-C1 Q1 86.2%, Fig. 6E IgGFc-SH1990-C3 Q1 84.7%) but are essentially not activated (Fig. 6C, CD8-SH1990-C1 Q2 2.1%, Fig. 6E IgGFc-SH1990-C3 Q2 0.32%). Fig. 6D shows T-cell hybridoma cells expressing the CAR having a structure according to Fig. 3 with a CD8 hinge and CD8 TM domain containing the scFv encoded by SEQ ID NO: 25 (CD8-SH1990-C1), resulting in an activation in the presence of cells expressing the target antigen (Fig. 6D, Q2 23.8%). Fig. 6F for the CAR having a structure according to Fig. 4, with an IgG hinge and CD4 TM domain containing an scFv encoded by SEQ ID NO: 27 (IgGFc-SH1990-C3) shows that also in this embodiment the T-cells are specifically activated by presence of the HEK293-T-cells expressing the target antigen. These results show that both the embodiment of the CAR containing an IgG hinge with the CD4 TM domain and the embodiment of the CAR containing a CD8 hinge with the CD8 TM domain are effectively expressed in T cells and are specifically activated in response to presence of human MBP when expressed by a cell, for each of the scFv.

The hybridoma T-cells expressing a CAR of the invention were also target antigen-specifically activated by HEK-293 T-cells expressing hMBP. Fig. 6A and 6B show hybridoma T-cells devoid of the CAR (untransduced) in presence of HEK-293 T-cells (HEK-293T) which do not express MBP (Untransfected) as very low to absent unspecific background. Fig. 6C and 6D for the embodiment CD8-SH1990-C1 of the CAR show expression of the CAR (Q1 86.2%) CAR-expressing cells with activated background (Q2 2.10%) and in Fig. 6D specific activation of CAR-expressing cells (Q2 23.8%), both of which are comparable to background and activation with plate-bound target antigen of Fig. 5C and Fig. 5D. For the embodiment of IgGFc-SH1990-C3 of the CAR, Fig. 6E shows high expression of the CAR (Q1 84.7%) and very low background non-specific activation by non-stimulating HEK-293 T-cells (Q2 0.32), and Fig 6F for stimulation by HEK-293 T-cells expressing the target antigen hMBP shows that also this scFv induces target antigen-specific activation (Q2 24.2%, CAR expression only in Q1 67.7%) of T-cells expressing the CAR.

Further, these results show that both the embodiment of the CAR containing the CD8 hinge with the CD8 TM domain (generally indicated by CD8 in the designation of a CAR) and the embodiment of the CAR containing the ΔFcIgG hinge with the CD4 TM domain (generally indicated by IgGFc in the designation of a CAR) when expressed in a Treg result in a MBP-specific activation of the Treg.

Further, the results show that each of these scFv embodiments is specific for human MBP.

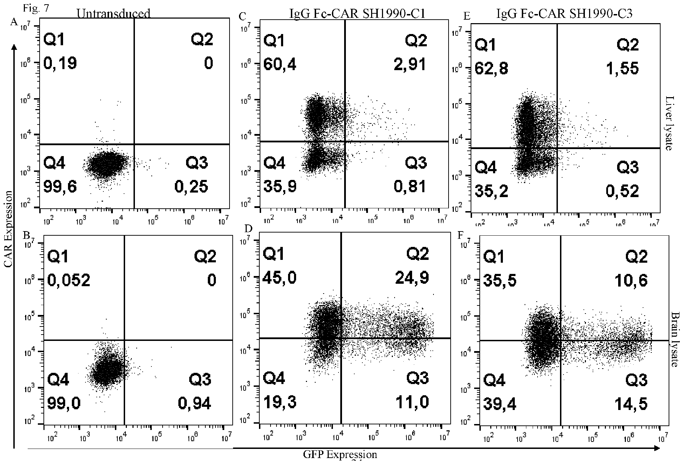

In an assay for testing target tissue specificity, the hybridoma T-cells expressing a CAR of the invention were contacted with murine liver lysate or with murine brain lysate. Activation of the hybridoma T-cells by the CAR due to presence of target antigen was detected as GFP expression. Fig. 7A and Fig. 7B show background for the hybridoma T-cells without expression of CAR (Untransduced), background of detection of the CAR (Fig. 7A Q1 0.19%, Fig. 7B Q1 0.052%) with activation of 0% in Q2.

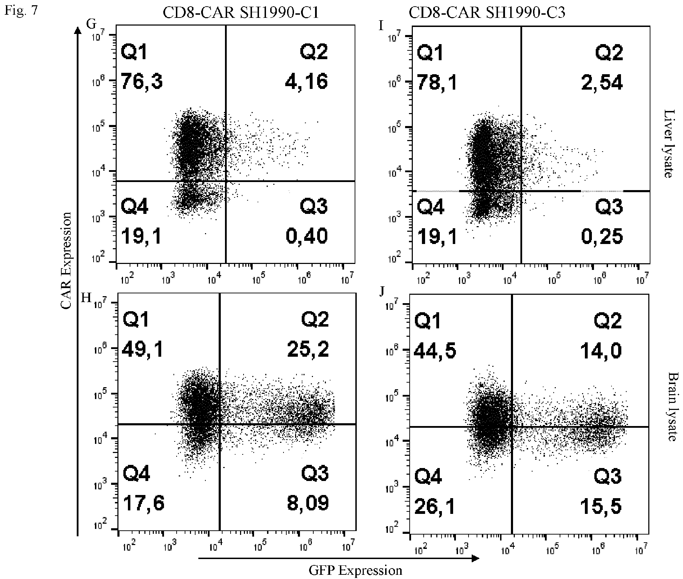

Results of the hybridoma T-cells expressing a CAR containing the scFv SH1990-C1 in the embodiment containing the ΔFcIgG hinge with the CD4 TM domain (IgGFc-CAR according to Fig. 1) are shown in Fig. 7C (Q2 2.91%) and Fig. 7D (Q2 24.7%), for the embodiment of the CD8 hinge with the CD8 TM domain in Fig. 7G (Q2 4.16%) and Fig. 7H (Q2 25.2%) for the CAR containing the scFv encoded by SEQ ID NO: 25, and in the embodiment containing the ΔFcIgG hinge with the CD4 TM domain in Fig. 7E (Q2 1.55%) and Fig. 7F (Q2 10.6%), and in the embodiment containing the CD8 hinge with the CD8 TM domain (CD8-CAR according to Fig. 2) in Fig. 7I (Q2 2.54%) and Fig. 7J (Q2 14.0%) for the CAR containing the scFv encoded by SEQ ID NO: 25, both indicating specific activation by brain lysate only.

Results of the hybridoma T-cells expressing a CAR containing the scFv SH1990-C3 in the embodiment containing the ΔFcIgG hinge with the CD4 TM domain (IgGFc-CAR) are shown in Fig. 7E (Q2 1.55%) and 7F (Q2 10.6%), and in the embodiment containing the CD8 hinge with the CD8 TM domain (CD8-CAR) in Fig. 7I (Q2 2.54%) and Fig. 7J (Q2 14.0%), both indicating specific activation by brain lysate only.

### Example 2: Homing of T-cells expressing a CAR to brain in EAE mice

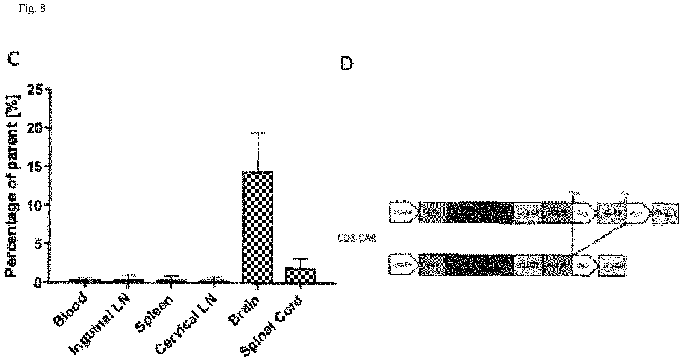

As an animal model of MS, C57BL/6 mice bearing EAE were used. EAE was induced in C57BL/6 mice by immunization with MOG35-55-peptide. In this preliminary test, CAR-T-cells (Fox⁻) without regulatory phenotype were used, which accordingly attack MBP-bearing cells. The CAR-T-cells were generated by transfecting T-cells with a viral vector which did not express FoxP3, as schematically shown in Fig. 8D by the excision of the P2A site and of the FoxP3 encoding section. At the peak of the MS-like disease, 1 × 10⁶ generated CAR-T-cells (Fox⁻) expressing an anti-MBP-CAR (CD8-SH1990-C3) or a non-specific CAR were adoptively transferred into these EAE mice. Three days later organs were harvested and analyzed using flow cytometry by staining for the reporter gene Thy1.1.

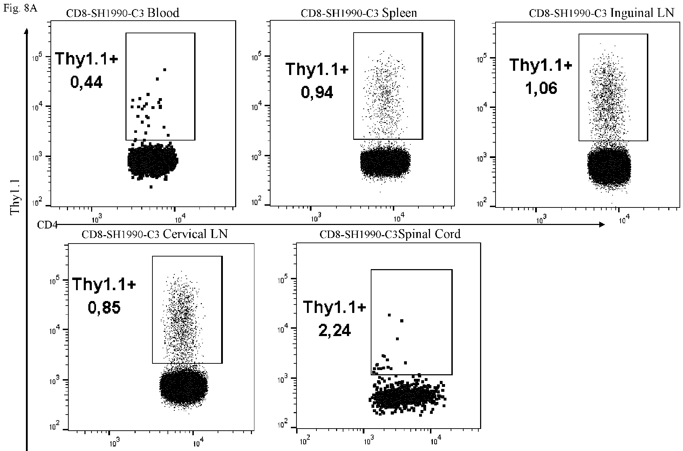

Fig. 8A shows representative results of the flow cytometric analysis of CD4+ cells gated on CD4 and Thy1.1. of different organs as indicated. Depicted are the dot plots of EAE mice treated with MBP-specific (CD8-SH1990-C3) CAR-T-cells in a 3-day homing assay. For blood (Blood), spleen (Spleen), and inguinal lymph nodes (Inguinal LN), a normal distribution of CAR-bearing T-cells is found, and also in the cervical lymph node (Cervical LN) only a very low percentage (0.35%) of CAR-bearing T-cells is found. In contrast, in spinal cord (Spinal Cord, individual dots are thicker for clarity) an increased percentage of CAR-bearing T-cells of 2.24% is found.

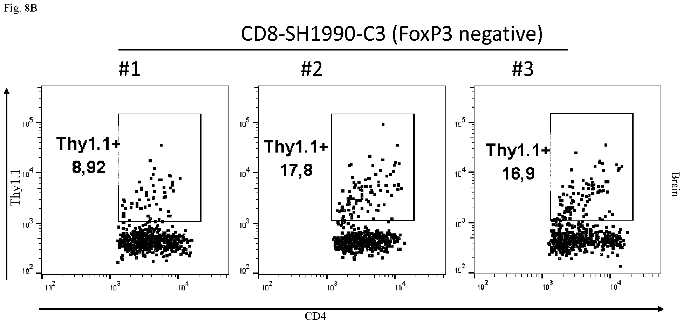

In Fig. 8B dot plots show for all of the three treated mice (#1 8.92%, #2 17.8%, #3 16.9%) in the respective brain sample gated on CD4 (X-axis) and Thy 1.1 (Y-axis) that the T-cells expressing a CAR of the invention preferentially migrate to and are activated in the brain. The bar graphs of Fig. 8C show the percentage of Thy 1.1+ from the CD4+ population in the indicated tissue, showing that the T-cells expressing a CAR of the invention preferentially migrate to and are activated in the brain.

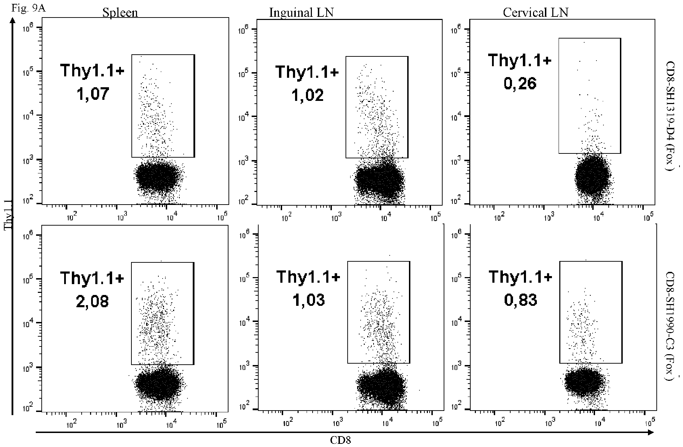

In a further preliminary test, CAR-T-cells (Fox⁻) without regulatory phenotype expressing a CAR specific for MBP (CD8-SH1990-C3) was used, and in comparison CAR-T-cells (Fox⁻) expressing a phycoerythritin-specific CAR (anti-PE-CAR) as a non-specific control (CD8-SH1319-D4) containing the same mCD8 hinge and mCD8 TM domains as well as the same intracellular signaling domains. Fig. 9A, flow cytometry of CD8+ T-cells gated for CD8 and Thy1.1, for the comparative CD8-SH1319-D4 shows background activation, and for the CAR specific for MBP shows comparative low activation in spleen (Spleen), inguinal lymph nodes (Inguinal LN), and in cervical lymph nodes (Cervical LN).

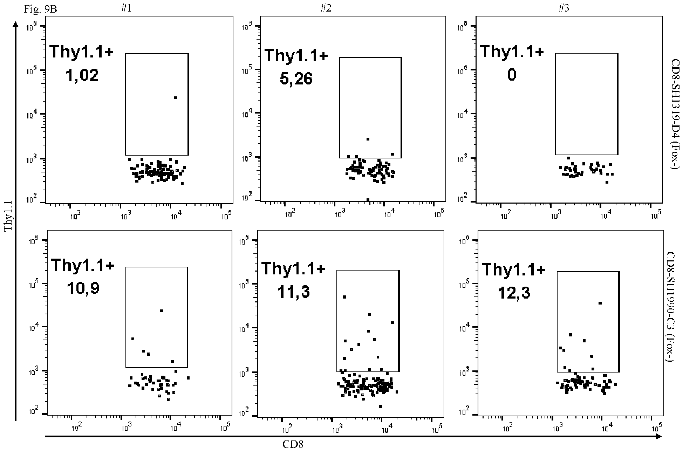

Fig. 9B shows dot plots of flow cytometry analyses for brain homogenate of the three mice (#1, #2, #3) gated for CD8 and Thy 1.1, showing that of the CAR-T-cells expressing the anti-MBP-CAR a higher proportion migrated into the brain than of the CAR-T-cells expressing the non-specific anti-PE-CAR.

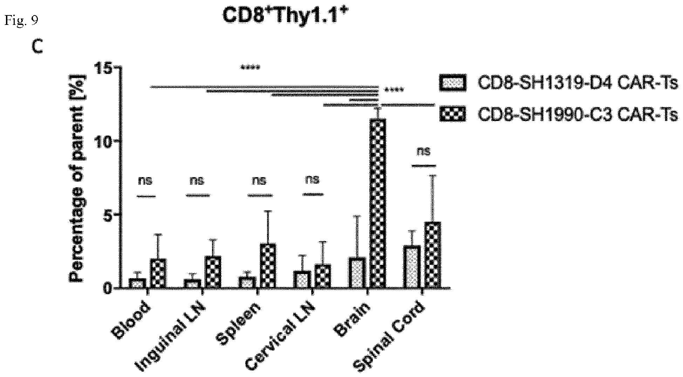

Fig. 9C shows a statistical analysis of the percentage of Thy 1.1+ T-cells from the CD8+ population. P values (*, **, *** and ****, indicate P < 0.05, P < 0.01, P <0.001 and P < 0.0001, respectively. ns: not significant) were calculated by a two-way ANOVA with Sidak's multiple comparison test. Error bars indicate the SD. The results shows a significantly high proportion of T-cells bearing the CAR of the invention (CD8-SH1990-C3 CAR-Ts) had migrated into brain tissue as well as into spinal cord and in other tissues was present only at normal distribution levels, whereas the comparative CAR (CD8-SH1319-D4 CAR-Ts) resulted in normal distribution in all tissues.

### Example 3: Homing of T-cells expressing a CAR to brain in TMEV-IDD mice

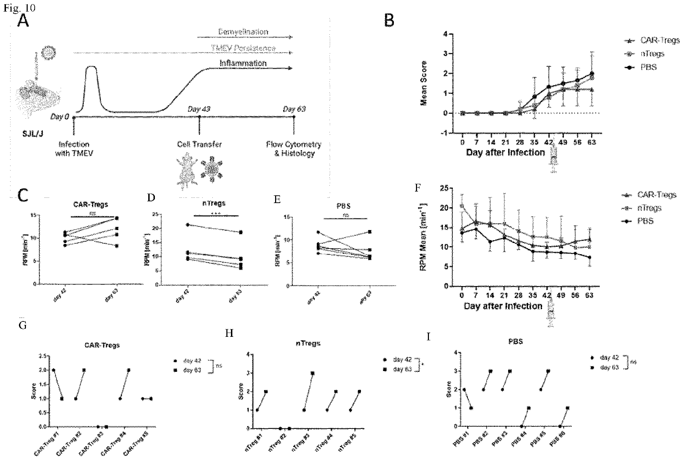

As an animal model of MS, C57BL/6 mice bearing the Theiler's murine encephalitis virus-induced demyelinating disease (TMEV-IDD) were used. Fig. 10A shows a schematic timeline of the TMEV-IDD induction with adoptive cell transfer at day 43 (i.p., 2 × 10⁵ CAR-Tregs or nTregs, or PBS), and endpoint analysis at day 63. While the TME virus persists in the SJL mice, chronic inflammation and demyelination start around day 30 after MS-like disease induction. Mice were weekly scored for their health, a low score indicating good health, a higher score indicating a worse health state, and tested on their Rotarod performance. Fig. 10B shows mean values for the three treatment groups of mice having received MBP-specific CAR-Tregs (CD8-SH1990-C3), having received nTregs, or PBS as a negative control. Error bars indicate the SD. The results shows that administration of the CAR-Tregs of the invention has an ameliorating effect on health, indicating a therapeutic effect of the CAR-Tregs of the invention in the treatment of the model MS. Fig. 10C for CAR-Tregs of the invention, Fig. 10D for natural Tregs (nTregs), Fig. 10E with PBS as a negative control show the Rotarod performances of individual mice at day 42 and at day 63, indicating improved performance only for CAR-Tregs, deteriorated performance for nTregs, and deteriorated performance for the negative control, except for one mouse which improved its performance. Fig. 10F gives an overview of the Rotarod performance over the duration of the experiment, showing an improvement of the performance subsequent to administration of the Tregs expressing the CAR of the invention only, better than by administration of nTregs or negative control (PBS). Fig. 10G, for CAR expressing Tregs of the invention, Fig. 10H for nTregs, and Fig. 10I for PBS as negative control for individual mice show the clinical scores between treatment start (day 42) and experiment end (day 63). Depicted are all mice of each treatment group individually. P values (*, **, *** and ****, indicate P < 0.05, P < 0.01, P <0.001 and P < 0.0001, respectively. ns: not significant) were calculated by a two-way ANOVA with Sidak's multiple comparison test. These results show that administration of Tregs expressing a CAR of the invention in one individual resulted in improvement of the health status (from 2.0 to 1.0), for 2 individuals maintained the health status (from 0 to 0 , respectively from 1.0 to 1.0) and in 2 individuals was accompanied by deterioration of the health status from a score of 1.0 to a score of 2.0, whereas administration of nTregs only resulted in maintaining the health status of 0 in one individual only and in a deterioration in 4 individuals (from 1 to 2 in 3 individuals, from 1 to 3 in one individual), and negative control (Fig. 10I) in one individual showed improvement of the health status (from 2 to 1), and in 5 individuals showed deterioration (from 2 to 3, or from 0 to 1).

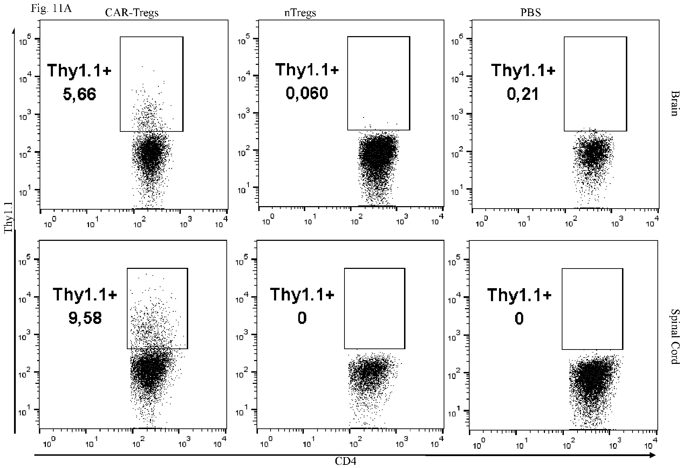

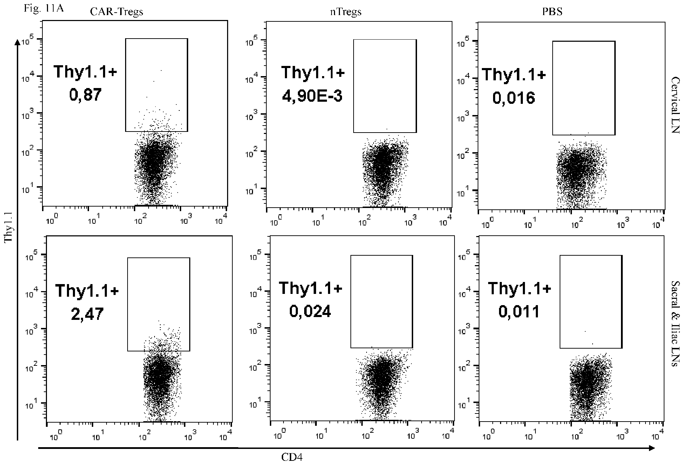

Fig. 11A shows low cytometric analyses of different organs from the therapeutic experiment in the TMEV-IDD experiment. As a representative example, the flow cytometric analyses of different organs is shown. Depicted are the dot plots of TMEV-IDD mice treated with either MBP-specific CAR-Tregs (CD8-SH1990-C3), nTregs, or PBS (negative control) at 3 weeks after administration. The dot plots show the CD4+ cells gated on CD4 and Thy1.1.

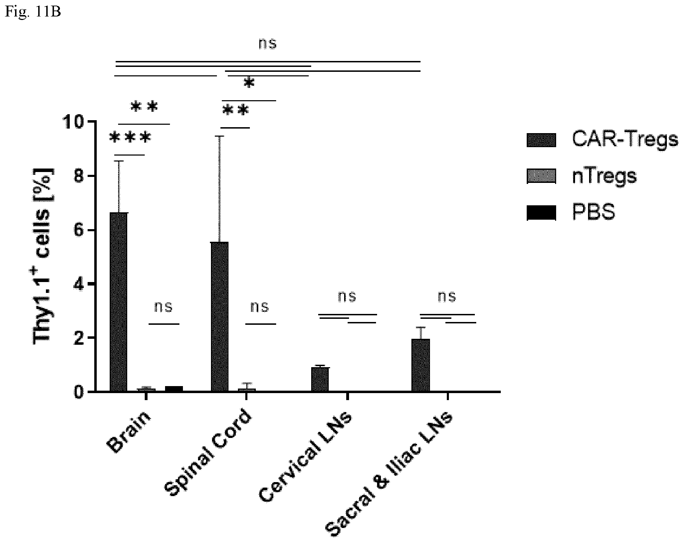

Fig. 11B in the statistical analysis of the percentage of Thy 1.1+ from the CD4+ quantifies the data shown for some mice analysed according to Fig. 11A. P values (*, **, *** and ****, indicate P < 0.05, P < 0.01, P <0.001 and P < 0.0001, respectively. ns: not significant) were calculated by a two-way ANOVA with Sidak's multiple comparison test. For each location, the left bars indicate MBP-CAR-Tregs, the middle graphs show nTregs which show very low background in brain and spinal cord and absent in cervical lymph nodes and in sacral and iliac lymph nodes, the right bars show PBS, which show absence for spinal cord, for cervical lymph nodes (Cervical LNs), and for sacral and iliac lymph nodes (Sacral & Iliac LNs). Error bars indicate the SD. These results show that Treg cells expressing a MBP-specific CAR of the invention are present essentially only in brain and spinal cord.

### Example 4: Expression of MOG-specific CAR and marker protein and MOG-specific activation of T-cells

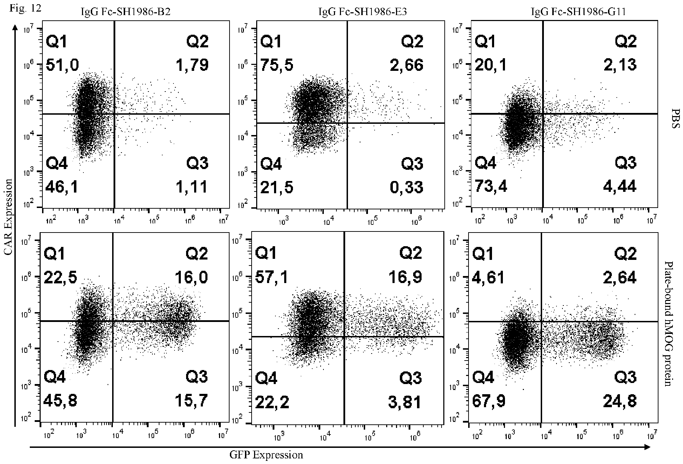

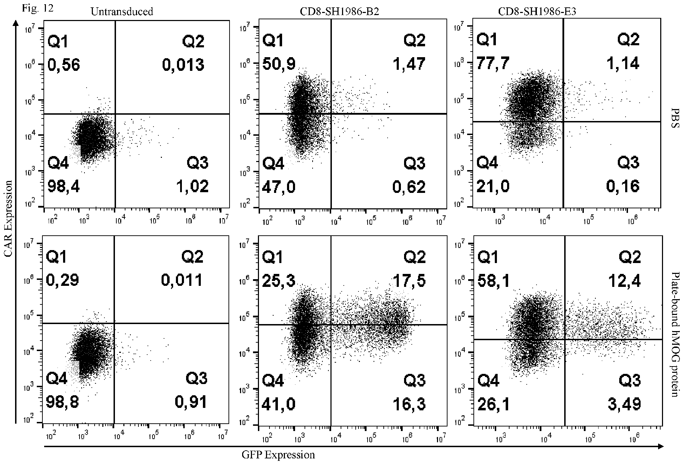

As described in Example 1 for MBP-specific CARs, several MOG-specific CARs with the same signal peptide, hinge and TM domain and intracellular signaling domains were used, containing one of the scFv encoded by SEQ ID NO: 33 (SH1986-B2), SEQ ID NO: 35 (SH1986-E3), SEQ ID NO: 37 (SH1986-G11) were expressed in hybridoma T-cells and tested for activation of the T-cells by plate-bound human MOG (hMOG). Results are depicted in Fig. 12A and Fig. 12B for T-cells without CAR (Untransduced), showing background only, and for the IgGFc-CAR containing the scFv SH1986-B2 in Fig. 12C as negative control (PBS) and in Fig. 12D with induction (Plate-bound hMOG protein) (Q2 16%), for the CD8-CAR in Fig. 12E as negative control and Fig. 12F with induction (Q2 17.5%). For the IgGFc-CAR containing the scFv SH1986-E3 Fig. 12G shows background, Fig. 12H shows induction (Q2 16.9%), for the CD8-CAR in Fig. 12I as negative control and Fig. 12J with induction (Q2 12.4%). For the IgGFc-CAR containing the scFv SH1986-G11, Fig. 12K shows background and Fig. 12L shows induction (Q2 2.64%).

The results show that also the CARs containing a MOG-specific scFv target-antigen specifically activate T-cells both as IgGFc-CAR and as CD8-CAR.

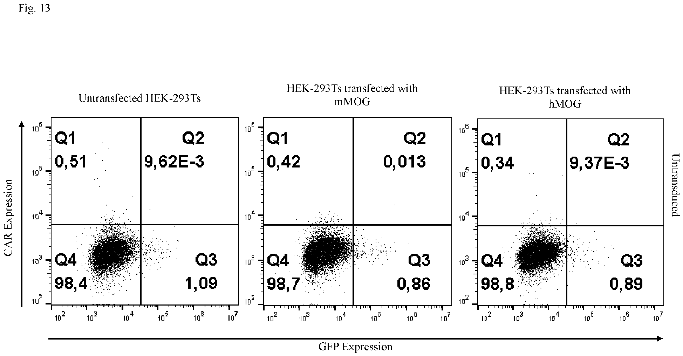

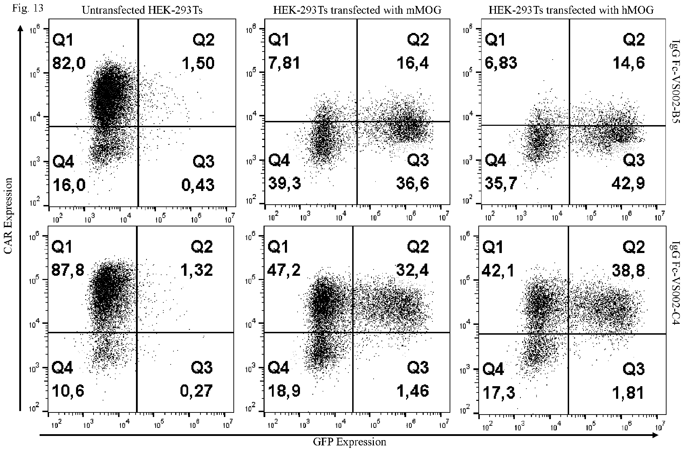

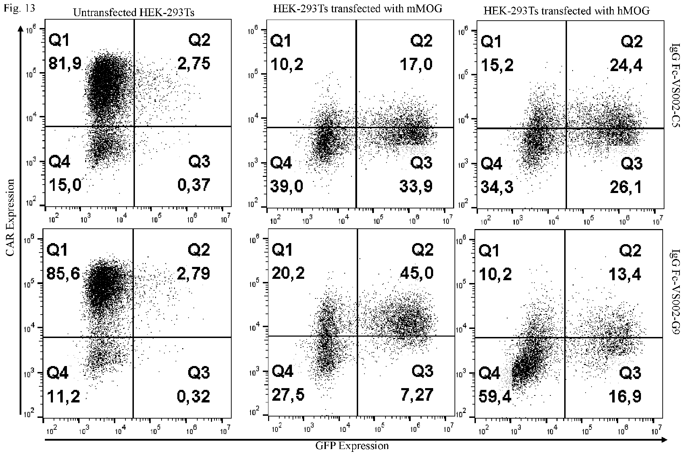

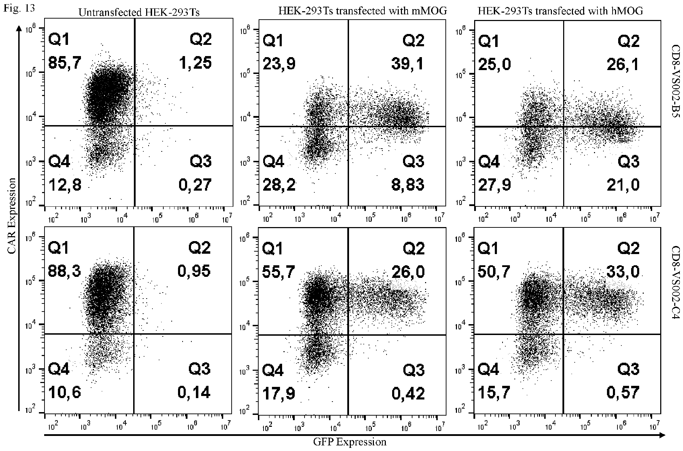

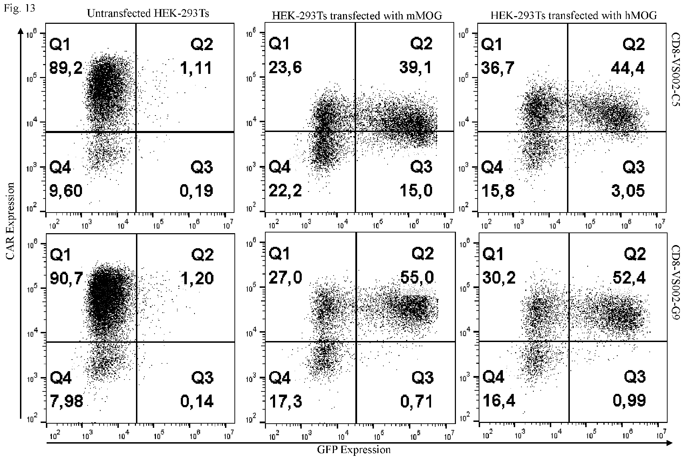

Induction of hybridoma T-cells expressing an IgGFc-CAR or CD8-CAR containing an scFv specific for MOG is shown in Fig. 13, wherein HEK-293 T-cells were transduced to express murine MOG (HEK-293Ts transfected with mMOG, Fig. 13, middle row) or human MOG (HEK-293Ts transfected with hMOG, Fig. 13, lower row), with controls by untransfected HEK-293 T-cells (Untransfected HEK-293Ts, Fig. 13, upper row).

The results show that the CARs specific for MOG result in target-antigen (MOG) specific activation of T-cells induced by the presence of cells expressing either human MOG or murine MOG.

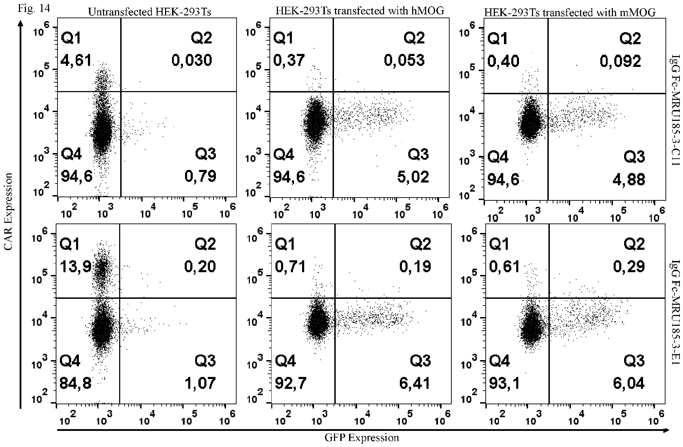

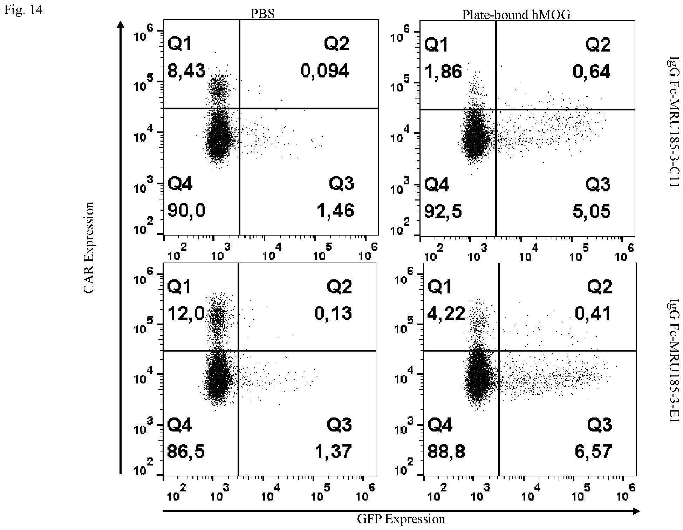

Fig. 14 shows cytometry results for activation of hybridoma T-cells expressing an IgGFc-CAR of the invention containing the scFv MRU185-3-C11 or the scFv MRU185-3-E1. As indicated by GFP-expression, the T-cells are activated by plate-bound human MOG (hMOG), by HEK293-T-cells expressing human MOG (HEK-293Ts transfected with hMOG), and by HEK293-T-cells expressing murine MOG (HEK-293Ts transfected with mMOG), in comparison to negative controls using untransfected HEK293-T-cells or PBS.

### Example 5: Homing of T-cells expressing a CAR to brain in EAE mice

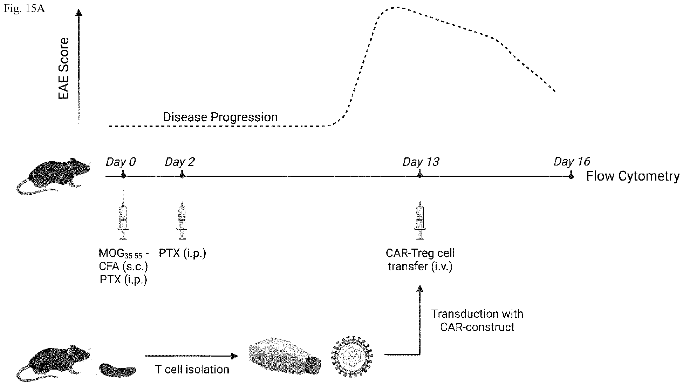

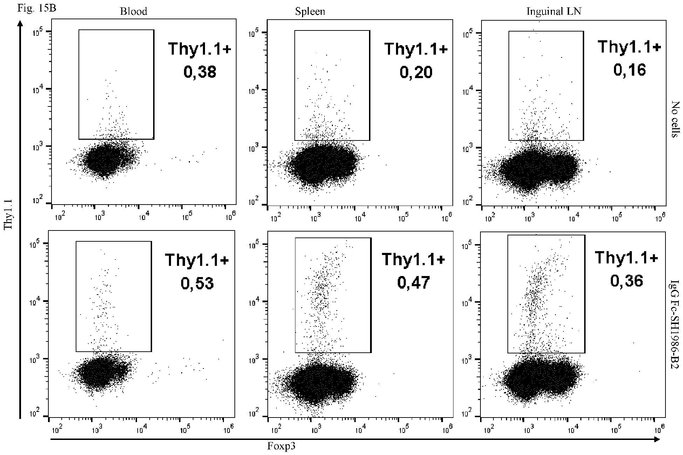

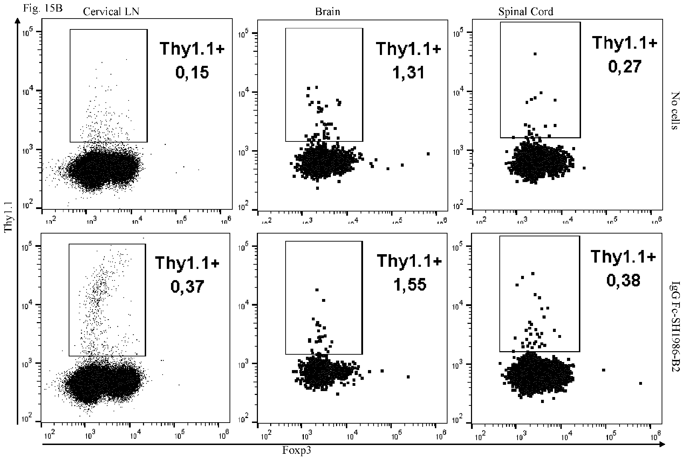

As described in Example 2, as an animal model of MS, C57BL/6 mice bearing EAE were used and analysed by adoptive transfer of 1×10⁶ murine Treg cells as schematically depicted in Fig. 15A. Prior to the transfer, the Treg cells were ex vivo transduced to express a MOG-specific CAR (IgGFc-CAR containing the scFv SH1986-B2). Organ-specific migration was analysed by flow cytometry. The results as depicted in Fig. 15B in comparison to background (No cells) by way of the exemplary scFv show that Treg cells expressing the MOG-specific CARs of the invention specifically migrate into brain.

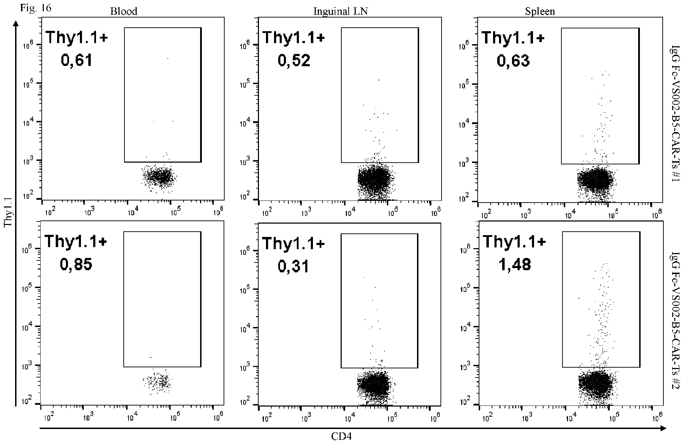

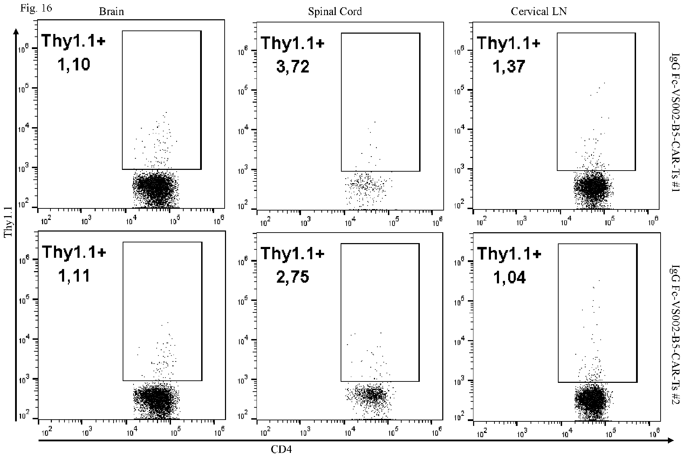

Fig. 16 shows the results of two individual mice (#1 and #2) after adoptive transfer of Treg cells expressing an IgGFc-CAR containing the scFv VS002-B5. In both individuals, the Treg cells specifically migrate into brain.

Due to the immune suppressive activity of regulatory T-cells (Treg) expressing a CAR of the invention, which activity is specifically induced by MBP or MOG, and wherein the Treg migrate into the brain, these CARs and Treg expressing one of these CARs are suitable for use in the treatment of MS and/or AD.

## Claims

1. CAR for use in the treatment of multiple sclerosis and/or Alzheimers disease, the CAR containing from N-terminus to C-terminus an scFv portion, a hinge, a transmembrane domain and at least one signaling domain, wherein the scFv portion is specific for brain and/or for spinal cord **characterized in that** the scFv portion contains a pair of CDR3 selected from
- amino acids No. 97..105 of SEQ ID NO: 29 and amino acids No. 92..100 of SEQ ID NO: 30, or
- amino acids No. No. 97..111 of SEQ ID NO: 31 and amino acids No. 90..100 of SEQ ID NO: 32, or
- amino acids No. 97..111 of SEQ ID NO: 51 and amino acids No. 91..100 of SEQ ID NO: 52, or
- amino acids No. 97..115 of SEQ ID NO: 53 and amino acids No. 88..98 of SEQ ID NO: 54, or
- amino acids No. 97..113 of SEQ ID NO: 55 and amino acids No. 94..102 of SEQ ID NO: 56, or
- amino acids No. 97..108 of SEQ ID NO: 57 and amino acids No. 88..108 of SEQ ID NO: 58, or
- amino acids No. 99..109 of SEQ ID NO: 59 and amino acids No. 90..100 of SEQ ID NO: 60, or
- amino acids No. 97..107 of SEQ ID NO: 61 and amino acids No. 92..102 of SEQ ID NO: 62, or
- amino acids No. of SEQ ID NO: 97..112 of SEQ ID NO: 63 and amino acids No. 92..100 of SEQ ID NO: 64, or
- amino acids No. of SEQ ID NO: 97..109 of SEQ ID NO: 65 and amino acids No. 88..98 of SEQ ID NO: 66, or
- amino acids No. 97..106 of SEQ ID NO: 67 and amino acids No. 89..97 of SEQ ID NO: 68.

2. CAR for use in the treatment of multiple sclerosis and/or Alzheimers disease according to one of the preceding claims, **characterized in that** in a regulatory T cell (Treg) the CAR is produced as a fusion protein with FOXP3 with a protease site arranged between the CAR and FOXP3.

3. CAR for use in the treatment of multiple sclerosis and/or Alzheimers disease according to one of the preceding claims, **characterized in that** the CAR is encoded by a nucleic acid construct which downstream from the CAR encoding section contains a sequence encoding deltaLNGFR or CD20 epitope as a marker, under the control of an IRES.

4. CAR for use in the treatment of multiple sclerosis and/or Alzheimers disease according to one of the preceding claims, **characterized in that** the scFv comprises a pair of variable chains selected from SEQ ID NO: 29 and SEQ ID NO: 30, SEQ ID NO: 31 and SEQ ID NO: 32, SEQ ID NO: 51 and SEQ ID NO: 52, SEQ ID NO: 53 and SEQ ID NO: 53, or SEQ ID NO: 55 and SEQ ID NO: 56, or SEQ ID NO: 57 and SEQ ID NO: 58, or SEQ ID NO: 59 and SEQ ID NO: 60, or SEQ ID NO: 61 and SEQ ID NO: 62, or SEQ ID NO: 63 and SEQ ID NO: 64, or SEQ ID NO: 65 and SEQ ID NO: 66, or SEQ ID NO: 67 and SEQ ID NO: 68.

5. CAR for use in the treatment of multiple sclerosis and/or Alzheimers disease according to one of the preceding claims, **characterized in that** the CAR at the C-terminus of the scFv portion contains a hinge having an amino acid sequence of SEQ ID NO: 5 or of SEQ ID NO: 6.

6. CAR for use in the treatment of multiple sclerosis and/or Alzheimers disease according to one of the preceding claims, **characterized in that** the CAR at the C-terminus of the scFv portion contains a hinge and a transmembrane domain having an amino acid sequence of SEQ ID NO: 7 or of SEQ ID NO: 8.

7. Regulatory T-cell for use in the treatment of multiple sclerosis and/or Alzheimers disease, the regulatory T-cell expressing a CAR, **characterized in that** the regulatory T cell is genetically manipulated to express a CAR according to one of the preceding claims.

8. Regulatory T-cell for use in the treatment of multiple sclerosis and/or Alzheimers disease according to claim 7, wherein the T cell is immunologically compatible with the patient.

9. Regulatory T-cell for use in the treatment of multiple sclerosis and/or Alzheimers disease according to one of claims 7 to 8, **characterized in that** the T cell in the presence of brain tissue has immune suppressive activity.

10. In vitro process for producing a regulatory T-cell according to one of claims 7 to 9, **characterized by** introducing into a T-cell a nucleic acid construct encoding a CAR according to one of claims 1 to 6.
